# EUROPEAN PATENT APPLICATION

(11) **EP 4 516 401 A1**
(43) Date of publication of application: **05.03.2025**
(21) Application number: 23725556.7
(22) Date of filing: 16.03.2023
(51) Int. Cl.: B01L 3/00

(54) **APPARATUS AND METHOD FOR SEPARATING TARGET OBJECT**

(30) Priority: 28.04.2022 KR 20220052701
(71) Applicant: Curiosis Co., Ltd., Seoul 06221 (KR)
(72) Inventor: YUN, Ho Young, Seoul 06221 (KR); KANG, Myeong Woo, Seoul 06221 (KR); SHIN, Tae Hwan, Seoul 06221 (KR); LEE, Yu Jin, Seoul 06221 (KR); SHIN, Sung Gyu, Seoul 06221 (KR)
(74) Representative: Kuhnen & Wacker Patent- und Rechtsanwaltsbüro PartG mbB
(86) International application number: PCT/KR2023/003496
(87) International publication number: WO 2023/210966

(57) **Abstract**

An apparatus for separating a target object is provided. The apparatus includes an injection unit into which a fluid including microparticles is injected, a passage unit allowing a flow of the target object to be concentrated in a predetermined direction during a process of the injected fluid flowing, the passage unit comprising a plurality of engraved structures having a groove shape in a direction perpendicular to a main flow direction of the fluid, and a target object obtaining unit for obtaining the target object concentrated in the predetermined direction. At least one of the injection unit, the passage unit or the target object obtaining unit comprise a pillar structure arranged in an area other than the plurality of engraved structures.

## Description

### BACKGROUND

### 1. Field

The present disclosure relates to an apparatus and method for separating a target object. More specifically, the present disclosure relates to an apparatus and method for separating a target object smoothly by arranging a groove, a structure, etc. inside the apparatus.

### 2. Description of Related Art

Sample preparation technology for separating and concentrating microparticles such as cells or plasma plays a very important role in various fields such as biological research, in vitro diagnosis, treatment, and pharmaceuticals. In order to separate and concentrate these certain microparticles or plasma, a centrifuge which separates and concentrates the microparticles or plasma mainly through the difference in density between cells is used. Although the centrifuge is useful when processing a large amount of samples, the centrifuge has limitations in processing a small amount with high efficiency, is an expensive device, and has a risk of physically damaging microparticles or plasma. Accordingly, a microfluidic chip-based technology for separating and concentrating microparticles or plasma has recently been developed. This is a technology for separating and concentrating microparticles or plasma by installing an arbitrary structure capable of manipulating the flow in a channel of tens of micrometers to several millimeters. According to microfluidic chip-based technology for separating and concentrating microparticles or plasma, separation and concentration may be performed with less reagents and less power, the apparatus has high portability, and rapid analysis and detection may be performed with a low cost.

However, existing microfluidic chips have a problem that the center of the channel sags due to thermal deformation during the manufacturing process of the chip, and the cell concentration effect deteriorates due to reverse streamlines in some parts. Accordingly, there is a need for research to solve the sagging in the center of the channel and the deterioration in the concentration effect due to the reverse streamline, while performing excellent functions in separating and concentrating microparticles.

### Prior art reference

### Patent document

Korean Patent Laid-Open No. 2011-0005963 (laid-open date: January 20, 2011)

### Detailed description of invention

### Technical task

An embodiment of the present disclosure is to provide an apparatus and method for separating a target object, which have excellent performance in separating and concentrating the target object.

An embodiment of the present disclosure is to solve the sagging in the center of the channel of the microfluidic chip.

An embodiment of the present disclosure is to solve a phenomenon in which the concentration effect is reduced due to reverse streamlines.

### Summary

An embodiment of the present disclosure is to provide an apparatus and method for separating a target object, which have excellent performance in separating and concentrating the target object. An embodiment of the present disclosure is to provide an apparatus for separating a target object, comprising: an injection unit into which a fluid including microparticles is injected; and a passage unit allowing the flow of the target object to be concentrated in a predetermined direction during the process of the injected fluid flowing. The passage unit may comprise a plurality of engraved structures having a groove shape in a direction perpendicular to the main flow direction of the fluid. Also, the apparatus for separating the target object may further comprise a target object obtaining unit for obtaining the target object concentrated in the predetermined direction, and at least one of the injection unit, the passage unit or the target object obtaining unit may comprise a pillar structure arranged in an area other than the plurality of engraved structures.

In an embodiment, the apparatus for separating the target object may further comprise a non-target object discharging unit.

In an embodiment, the target object may be concentrated in a predetermined direction by a secondary flow generated in a direction perpendicular to the main flow direction of the fluid by the plurality of engraved structures.

In an embodiment, the apparatus for separating the target object may comprise a highway channel extending from at least a portion of the area between the injection unit and the target object obtaining unit.

In an embodiment, the plurality of engraved structures may not be arranged in the highway channel. For example, the plurality of engraved structures may be arranged in a passage unit other than the highway channel.

In an embodiment, the highway channel may be formed as a ditch-type channel in the depth direction on one side of the passage unit corresponding to a direction in which the microparticles are concentrated.

In an embodiment, the highway channel may have a width of 0.1% to 50% of the width of the passage unit.

In an embodiment, a micropattern formed by the plurality of engraved structures may have a curved shape.

In an embodiment, the plurality of engraved structures may be arranged on a bottom surface or a ceiling surface of the apparatus for separating microparticles, and the plurality of engraved structures may be formed being disconnected from each other.

In an embodiment, wherein the plurality of engraved structures may form a linear micropattern, and the linear micropattern may have an angle of 45 to 135 degrees with respect to the main flow direction of the fluid.

In an embodiment, the plurality of engraved structures may form a curved micropattern formed from a first point, which is the starting point, to a second point, which is the end point, and the tangent of the first point may have an angle of 45 to 135 degrees with respect to the main flow direction of the fluid, and the tangent of the second point may have an angle of 0 to 75 degrees or 105 to 180 degrees with respect to the main flow direction of the fluid.

In an embodiment, when the target object is a leukocyte, erythrocytes may be obtained in the non-target discharging unit, when the target object is plasma, blood cells may be obtained from the non-target discharging unit, and when the target object is a cell, a culture medium from which the cell is removed may be obtained in the non-target object discharging unit.

In an embodiment, the height of the pillar structure may correspond to the height of the passage unit, the pillar structure may have a pillar shape having a cross section of a circle, an ellipse, a streamline or a round polygon, and the maximum length of the cross-section of the pillar shape may be determined so that (height of the passage unit)/(maximum length of the cross-section) does not exceed a certain value.

In an embodiment, when there are a plurality of pillar structures, the distance between the plurality of pillar structures may be wider than or equal to the diameter of the microparticle to be obtained or the length of the engraved structure.

An embodiment of the present disclosure is to provide a method for separating a target object from a fluid using an apparatus for separating the target object, comprising: injecting a fluid including microparticles into an injection unit; and obtaining, at the target object obtaining unit, the target object concentrated in a predetermined direction during the process of flowing the fluid injected into the passage unit, wherein the passage unit comprises a plurality of engraved structures having a groove shape in a direction perpendicular to the main flow direction of the fluid, and at least one of the injection unit, the passage unit or the target object obtaining unit comprise a pillar structure arranged in an area other than the plurality of engraved structures.

In an embodiment, the method for separating the target object may further comprise obtaining a non-target object from a non-target object discharging unit.

In an embodiment, the target object may be concentrated in a predetermined direction by a flow generated in a direction perpendicular to the main flow direction of the fluid by the plurality of engraved structures.

In an embodiment, the apparatus for separating the target object may comprise a highway channel extending from at least a portion of the area between the injection unit and the target object obtaining unit.

### Effect of invention

According to an embodiment of the present disclosure, a target object may be separated and concentrated efficiently.

According to an embodiment of the present disclosure, it is possible to solve the sagging in the center of the channel of a microfluidic chip.

According to an embodiment of the present disclosure, it is possible to solve a phenomenon in which the concentration effect is reduced due to reverse streamlines.

According to an embodiment of the present disclosure, a micropattern, which is a curved inclined structure, is formed on the microfluidic chip. The curved inclined structure may greatly improve the flow rate of the secondary flow compared to the linear inclined structure, and the improved flow rate has an effect of greatly improving the separation performance for the plasma, cell or microparticle. In addition, by using the microfluidic chip according to the present disclosure having such effects, the usability is superior to the existing cell separating and concentrating technology, and cell damage may be minimized. Further, the microfluidic chip-based cell separating and concentrating technology has an effect of being applicable from an ordinary cell culture-based experiment process to a process for developing/producing stem cell and immune cell therapeutics.

### BRIEF DESCRIPTION OF THE DRAWINGS

Fig. 1A is a schematic view illustrating the constitution of the apparatus for separating a target object according to an embodiment of the present disclosure;
Fig. 1B is a partial enlarged view of the apparatus for separating the target object of Fig. 1A;
Fig. 1C is a view explaining the inclination of the micropattern of the apparatus for separating a target object of Fig. 1A;
Fig. 2A is a schematic view illustrating the constitution of the apparatus for separating a target object according to an embodiment of the present disclosure;
Fig. 2B is a partial enlarged view of the apparatus for separating a target object of Fig. 2A;
Fig. 2C is a view explaining the inclination of the micropattern of the apparatus for separating a target object of Fig. 2A;
Fig. 3 is a schematic cross-sectional view illustrating the apparatus for separating microparticles according to an embodiment of the present disclosure;
Fig. 4 is a schematic cross-sectional view illustrating the apparatus for separating microparticles comprising a highway channel according to an embodiment of the present disclosure;
Fig. 5 is a schematic cross-sectional view illustrating the apparatus for separating a target object in which sagging in the center of the channel occurs according to an embodiment of the present disclosure;
Fig. 6 is a schematic cross-sectional view illustrating the apparatus for separating a target object which solves the sagging in the center of the channel according to an embodiment of the present disclosure;
Figs. 7A to 7C are views illustrating a process of separating microparticles by a plurality of engraved structures having a groove shape according to an embodiment of the present disclosure;
Fig. 8 is a schematic view illustrating the arrangement and alignment of a plurality of engraved structures according to an embodiment of the present disclosure;
Fig. 9A is a drawing simulating the cell separation performance obtained from the micropattern having a linear shape according to an embodiment of the present disclosure;
Fig. 9B is a drawing simulating the cell separation performance obtained from the micropattern having a curved shape according to an embodiment of the present disclosure;
Figs. 10A and 10B are drawings showing the experimental results of cell separation performance obtained from the micropattern having a curved shape according to an embodiment of the present disclosure;
Fig. 11A is a view showing the separation performance of a target object in the case where there is no highway channel according to an embodiment of the present disclosure; and
Fig. 11B is a view showing the separation performance of a target object in the case where there is a highway channel according to an embodiment of the present disclosure.

### DETAILED DESCRIPTION

In order to clarify the technical idea of the present disclosure, embodiments of the present disclosure will be described in detail with reference to the accompanying drawings. In describing the present disclosure, if it is determined that a detailed description of a related known function or component may unnecessarily obscure the subject matter of the present disclosure, detailed description thereof will be omitted. Components having substantially the same functional configuration in the drawings are given the same reference numerals and symbols as much as possible, even if they are displayed on different drawings. For the sake of convenience in description, a device and method are described together, if necessary. Each operation of the present disclosure does not necessarily have to be performed in the order described, and may be performed in parallel, selectively, or separately.

The terms used in the embodiments of the present disclosure have been selected from general terms that are currently widely used as much as possible in consideration of the function in the present disclosure, but they may vary depending on the intention of a person skilled in the art or precedents, the emergence of new technologies, and the like. **In** addition, in a specific case, there may also be terms arbitrarily selected by the applicant, and in this case, the meanings thereof will be described in detail in the description of the corresponding embodiments. Therefore, the terms used in this specification should be defined based on the meanings of the terms and the overall content of the present disclosure, not simply based on the names of the terms.

Throughout the present disclosure, singular expressions may include plural expressions unless the context clearly specifies otherwise. Terms such as "include" or "have" are intended to designate that a feature, number, step, operation, component, part, or combination thereof exists, not to exclude the possibility of the presence or addition of one or more other features, numbers, steps, operations, components, parts or combinations thereof. **In** other words, throughout the present disclosure, when a certain part "includes" a certain component, it means that other components may be further included rather than excluding other components, unless otherwise specified.

The phrase "at least one of," when used with a list of items, means that different combinations of one or more of the listed items may be used, and only one item in the list may be needed. For example, "at least one of: A, B, and C" includes any of the following combinations: A, B, C, A and B, A and C, B and C, and A and B and C. In addition, terms such as "...unit" and "...module" described in the present disclosure refer to a unit that processes at least one function or operation, which may be implemented as hardware or software or a combination of hardware and software.

Throughout the present disclosure, when a part is "connected" to another part, this includes not only the case where a part is "directly connected" to another part, but also the case where it is "electrically connected" with another element in between. In addition, when a part "includes" a certain component, it means that other components may be further included rather than excluding other components, unless otherwise specified.

The expression "configured to" used throughout the present disclosure may be interchangeably used with terms such as "suitable for," "having the capacity to," "designed to," "adapted to," "made to," or "capable of" depending on the situation. The term "configured to" may not necessarily mean only "specifically designed to" in hardware. Instead, in some contexts, the expression "a system configured to" may mean that the system is "capable of" doing something in conjunction with other devices or components. For example, the phrase "a processor configured to perform A, B and C" may refer to a dedicated processor (e.g., embedded processor) for performing a corresponding operation, or a generic-purpose processor (e.g., CPU or application processor) capable of performing corresponding operations by executing one or more software programs stored in the memory.

As used in the present disclosure, the term "about" means within 10%, preferably within 5%, and more preferably within 1% of a given value or range.

An embodiment of the present disclosure is to provide an apparatus and method for separating a target object which aims at separating and/or concentrating specific microparticles such as cells or plasma in a desired direction.

According to an embodiment of the present disclosure, the apparatus for separating the target object may separate and/or concentrate plasma, cell or microparticles by inducing a secondary flow in a direction perpendicular to the fluid flow on the ceiling or bottom surface of the channel.

According to an embodiment of the present disclosure, the apparatus for separating the target object may greatly improve the flow rate of the secondary flow by using a curved inclined structure than when using a linear inclined structure. In addition, the separation performance for the target object may be improved due to the improved flow rate.

In addition, the apparatus for separating the target object according to an embodiment of the present disclosure has usability superior to existing apparatus for separating and concentrating microparticles, and may minimize damage to microparticles such as cells. Also, the apparatus and method for separating a target object according to an embodiment of the present disclosure may be applied in a variety of ways, from an ordinary cell culture-based experiment process to a process developing/producing stem cell and immune cell therapeutics.

An embodiment of the present disclosure may separate the target object in a desired direction by using a pattern shape of the apparatus for separating the target object. In addition, the target object may be separated in a certain direction inexpensively and conveniently through the pattern shape of the apparatus for separating the target object.

Throughout the present disclosure, the term "target object" means a target object to be separated through an apparatus for separating the target object, and may include, for example, microparticles, plasma, etc. Here, microparticles may include erythrocytes, platelets, leukocytes, circulating tumor cells, stem cells, effete stored erythrocytes, T-cells derived from autologous T-cell expansion, organic microparticles, inorganic microparticles, organic metallic microparticles, metallic microparticles, aerosol particles, bacteria, yeast, fungi, algae, viruses, microinvertebrates or their eggs, pollen, cell or tissue fragments, cell aggregates, cell debris (e.g., cell debris associated with DNA or RNA purification), bioreactor-produced cells or granules, proteins, protein aggregates, prions, vesicles, liposomes, precipitates (e.g., precipitates from blood or blood fractions, industrial process precipitates, wastewater precipitates, etc.), granules or cells from fermented food (e.g., granules or cells from fermented beverages), macromolecules, macromolecular aggregates, DNA, organelles, spores, gas bubbles, droplets and exosomes, and the like.

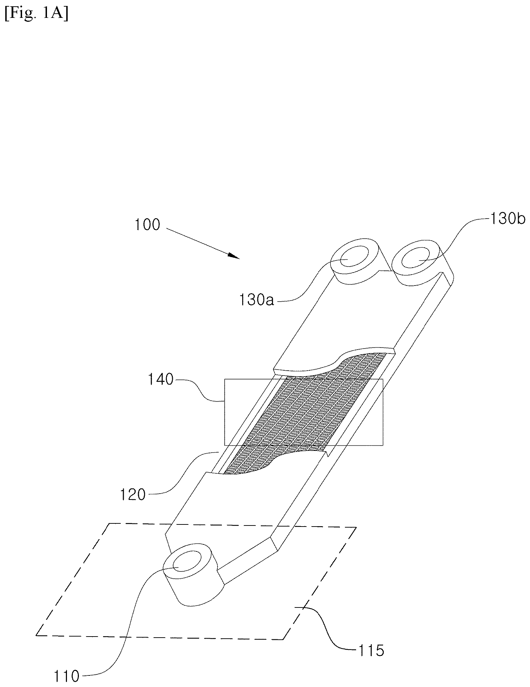

Fig. 1A is a schematic view illustrating the constitution of the apparatus for separating a target object according to an embodiment of the present disclosure.

Referring to Fig. 1A, the apparatus 100 for separating a target object may comprise an inlet 110, an injection unit 115, a passage unit 120, a target object obtaining unit 130a, a non-target object discharging unit 130b, etc. In an embodiment, a fluid including microparticles may be injected into the apparatus 100 for separating the target object through the inlet 110. The injection unit 115 may refer to a fluid flow passage around the inlet 110. In an embodiment, as the injected fluid flows through the injection unit 115 and the passage unit 120, the target object may be separated intensively in a certain direction. The separated target object may be concentrated in the target object obtaining unit 130a. Also, a non-target object that is not the target object may be concentrated in the non-target object discharging unit 130b.

Hereinafter, a process of injecting a fluid into the inlet 110 and concentrating a target object in the target object obtaining unit 130a will be described in more detail.

In an embodiment, a fluid including microparticles may be injected into the inlet 110. The fluid may be injected with, for example, a tube, a syringe, a pipette, etc. Also, for example, the fluid may include whole blood for the purpose of obtaining leukocytes or plasma. Or, the fluid may include cell culture media.

In an embodiment, while the injected fluid flows in the injection unit 115 and the passage unit 120, the target object may be separated in a certain direction. The target object obtaining unit 130a may be provided at the end of the apparatus 100 for separating a target object in a predetermined direction, to obtain the separated target object. Also, the non-target object discharging unit 130b may be provided at the end of the fluid passage of the apparatus 100 for separating a target object, to obtain the non-target object.

Alternatively, the target object obtaining unit may be provided at the end of the fluid passage of the apparatus 100 for separating a target object, and the non-target object obtaining unit (not illustrated) may be provided at the end of the apparatus 100 for separating a target object in a predetermined direction. For example, a channel may by formed such that the non-target object obtaining unit is provided in the middle of the fluid passage of the apparatus 100 for separating a target object. Also, a plurality of non-target object obtaining units may be provided.

For example, in the case where the apparatus 100 for separating a target object is an apparatus for separating plasma, when blood is injected into the inlet 110, plasma may be concentrated in the target object obtaining unit 130a, and blood cells (e.g., leukocytes, erythrocytes, platelets, etc.) may be concentrated in the non-target object discharging unit 130b. Accordingly, even when plasma is separated by the apparatus for separating plasma, blood cells, etc., may be used without being discharged.

Also, for example, in the case where the apparatus 100 for separating a target object is an apparatus for separating leukocytes, when blood is injected into the inlet 110, leukocytes may be extracted through the target object obtaining unit 130a, and erythrocytes may be extracted through the non-target object discharging unit 130b.

Also, for example, in the case where the apparatus 100 for separating a target object is an apparatus for separating cells, when a cell culture medium or a cell suspension is injected into the inlet 110, concentrated cells may be extracted through the target object obtaining unit 130a, and the culture medium in which specific cells are removed may be extracted through the non-target object discharging unit 130b.

In an embodiment, a target object of the apparatus 100 for separating a target object may vary depending on the fluid passage pattern of the apparatus 100 for separating a target object.

In an embodiment of the present disclosure, one target object obtaining unit 130a and one non-target object discharging unit 130b are illustrated. However, this is an exemplary embodiment, and at least one of the target object obtaining unit or the non-target object discharging unit can be a plurality. For example, the apparatus 100 for separating a target object may include two or more, e.g., four to ten, target object obtaining units. Also, in another embodiment, the apparatus 100 for separating a target object may include three or more, e.g., four to thirty, more specifically eight to fifteen, non-target object discharging units. The number of each of target object obtaining units and non-target object discharging units may be the same or different according to the target object.

Also, the target object obtaining unit 130a may be referred to as a first target object obtaining unit, and the non-target object discharging unit 130b may be referred to as a second target object obtaining unit. The constitution regarding the number of target object obtaining units 130a or non-target object discharging units 130b may be applied in the same manner to the target object obtaining unit and non-target object discharging unit of the apparatus for separating a target object according to each embodiment described below, although not specifically mentioned below.

In an embodiment, a plurality of channels may be formed in the apparatus 100 for separating the target object. The channel may be a passage extending from the inlet to the target object obtaining unit or non-target object discharging unit, and the number of channels may be determined to be the same or different depending on the target object. The number of channels may be one to forty, specifically two to thirty, more specifically five to twenty. For example, regarding the number of channels from the inlet to the target object obtaining unit, the apparatus for separating leukocytes may have six channels, the apparatus for separating plasma may have six channels, and the apparatus for separating cells may have two channels. However, these are exemplary embodiments, and the apparatus 100 for separating the target object may have various numbers of channels.

In an embodiment, each channel may start from the inlet and divide into several channels in the middle of the passage extending to target object obtaining units or non-target object discharging units, and accordingly additional channels may be formed. For example, separate channels may be formed in the middle of the channel and have non-target object discharging units at the ends thereof. For example, in the case where there are eight non-target object discharging units, eight additional channels may be formed from the channel(s) starting from the inlet.

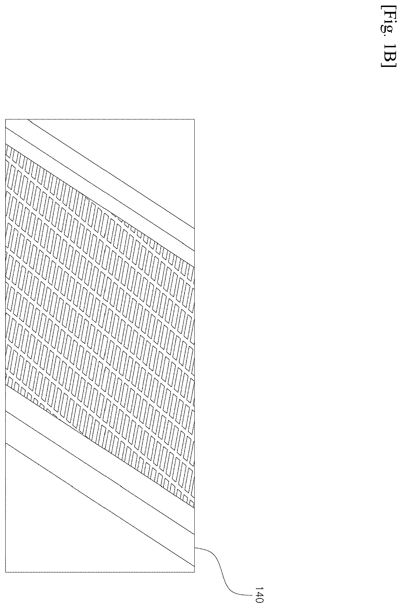

Fig. 1B is a partial enlarged view of the apparatus for separating a target object of Fig. 1A.

Referring to Fig. 1B, a portion 140 of the passage unit 120 of Fig. 1A is illustrated. In an embodiment, a target object may be separated using a certain pattern having an inclination at a predetermined angle relative to the main motion direction of the fluid in the apparatus 100 for separating the target object. The type, size, arrangement, etc., of pattern may be determined based on the main motion direction of the fluid and the certain direction in which the target object is to be separated. For example, with respect to the main flow direction of a fluid, the micropattern of the apparatus 100 for separating a target object may have an inclination inclined to the certain direction in which the target object is to be separated, and the inclination may include an inclination of 45 to 135 degrees with respect to the main motion direction of the fluid. When a fluid including microparticles is injected into the apparatus 100 for separating a target object, the inclination allows the fluid to move in the direction perpendicular to the inclined pattern groove, and accordingly the target object may be concentrated in a predetermined direction and flow.

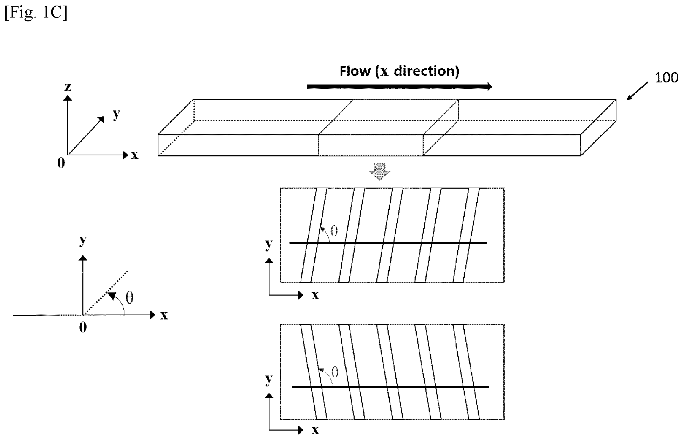

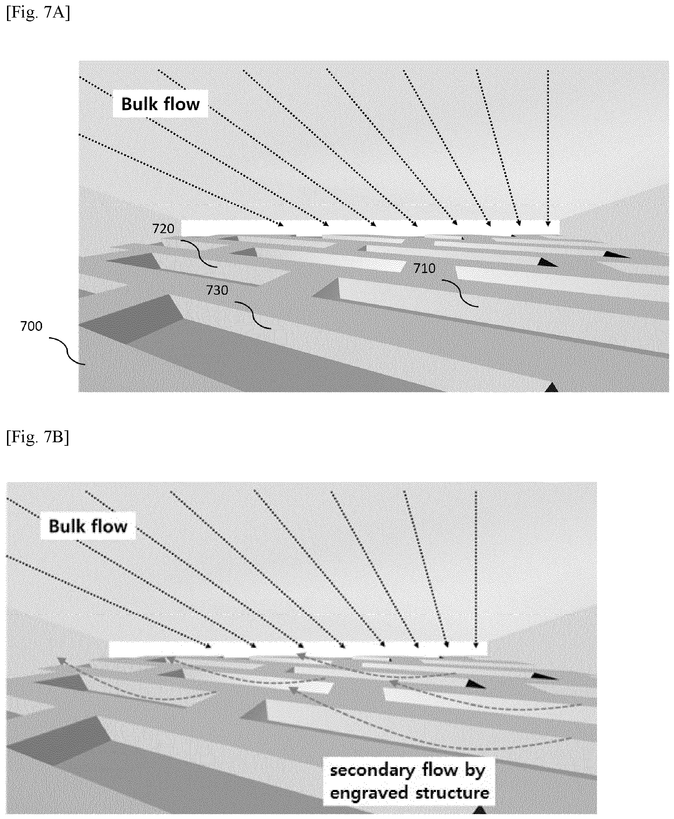

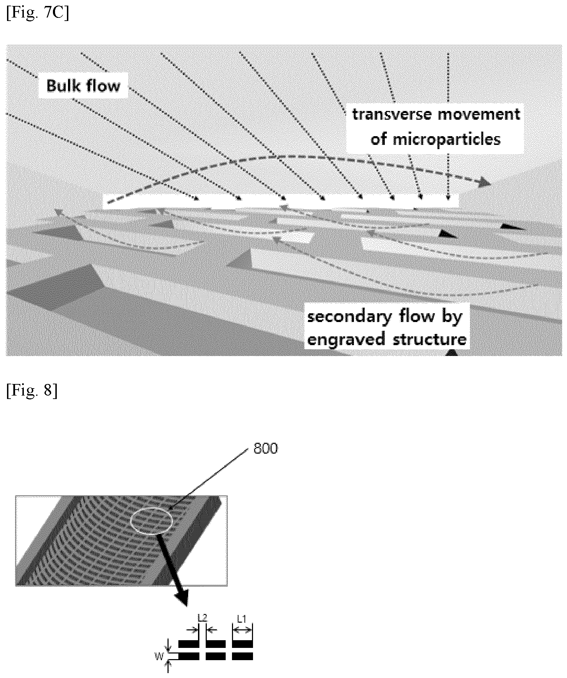

In an embodiment, the micropattern in the apparatus 100 for separating the target object may be formed by a plurality of grooves. The shape of the groove may be determined according to the type of target object to be separated. Here, the shape of the groove may include the height, width and length of the groove and the height of the passage unit 120, etc. For example, the height and width of the groove may be within one half to two times the diameter of the target object, the length of the groove may be within three times to ten times the diameter of the target object, and the height of the passage unit may be one time to three times the diameter of the target object. Here, the diameter of microparticles may include the average diameter of microparticles. In addition, the pattern in the passage unit 120 may include a pattern in which grooves are arranged at certain intervals. For example, the certain intervals may include about 1 to 70 µm (micrometer), for example about 5 to 40 µm, or about 10 to 20 µm. The groove may have a shape of a rectangle, diamond, triangle, ellipse, star, etc., but is not limited thereto. In an embodiment, the groove may be referred to as an engraved channel or an engraved structure. The target object may be separated by a plurality of engraved structures arranged on the ceiling or bottom surface of the channel in the passage unit 120. The engraved structure and the micropattern formed by the engraved structure will be described in detail referring to Fig. 7 and Fig. 1C, respectively.

In an embodiment, the laminar flow of the fluid allows the target object moved in a certain direction to maintain the position in the direction perpendicular to the passage unit 120 even in the area having no certain pattern. Accordingly, the fluid including the target object highly concentrated may be obtained through the target object obtaining unit 130a located at the end. Specifically, as the target object may be separated intensively in a predetermined direction by the passage unit 120 and flow, the target object obtaining unit 130a may be installed at the end in a predetermined direction to obtain the separated target object.

For example, the apparatus 100 for separating the target object having the target object obtaining unit 130a may separate leukocytes using whole blood. Specifically, leukocytes may be obtained at a high rate in the target object obtaining unit 130a using the separation flow process through the passage unit 120. As leukocytes are concentrated and moved to the target object obtaining unit 130a, erythrocytes may be obtained at a relatively high rate in the non-target object discharging unit 130b. The diameters of the target object obtaining unit 130a and the non-target object discharging unit 130b may be determined according to the target object and the non-target object, respectively. For example, in the case where the target object is leukocytes and the non-target object is erythrocytes, the diameter of the target object obtaining unit 130a may be greater than the diameter of the non-target object discharging unit 130b.

In an embodiment, the apparatus 100 for separating the target object may be manufactured from a polymer (polystyrene (PS), polycarbonate (PC), polymethylmethacrylate (PMMA), polydimethylsiloxane (PDMS), etc.). The apparatus may separate and arrange the target object even when the apparatus has a hydrophobic surface, but preferably, the apparatus has a hydrophilic surface considering the flow of a fluid.

Fig. 1C is a view explaining the inclination of the micropattern of the apparatus for separating a target object of Fig. 1A.

Referring to Fig. 1C, the micropattern of the apparatus 100 for separating a target object may be formed at an angle of 45 to 135 degrees with respect to the main flow direction of a fluid. For example, in the case where the main flow direction of the fluid is set to be on the x axis, the angle θ of the micropattern may range from about 45 degrees to about 135 degrees. Here, the angle θ of the micropattern may be determined according to the target object. For example, according to the target object, the angle θ of the micropattern in a channel of the apparatus for separating a target object may be about 45 degrees, and the angle θ of the micropattern in another channel may be about 135 degrees. In another embodiment, the angle θ of the micropattern in a channel may be about 60 degrees, and the angle θ in another channel may be about 120 degrees. In another embodiment, the angle θ of the micropattern in a channel may be about 75 degrees, and the angle θ in another channel may be about 105 degrees. However, these are exemplary embodiments, and the angle θ of the micropattern may be selected properly according to the target object.

In an embodiment, the micropattern may be formed by engraved structures of the apparatus 100 for separating a target object. For example, when a fluid including microparticles is injected into the apparatus 100 for separating a target object, the fluid moves in the direction perpendicular to the main flow direction of the fluid by the inclined engraved channel, and the target object may be concentrated in a predetermined direction and flow according to the angle of the micropattern.

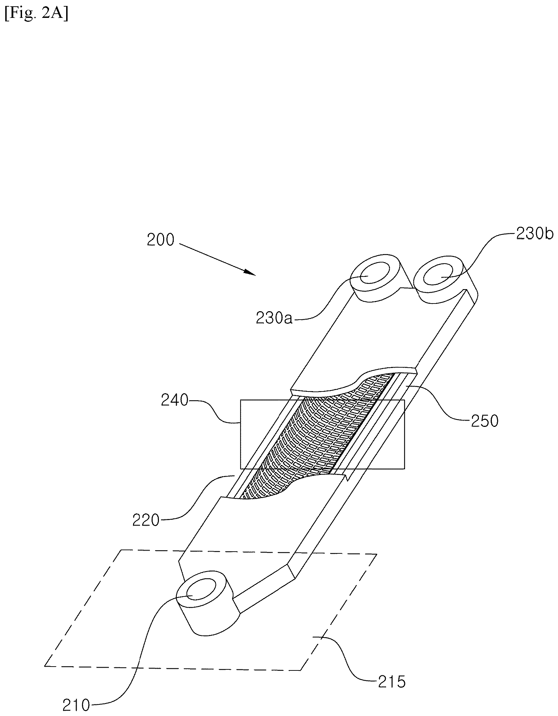

Fig. 2A is a schematic view illustrating the constitution of the apparatus for separating a target object according to an embodiment of the present disclosure.

Referring to Fig. 2A, the apparatus 200 for separating the target object further comprises a highway channel 250 and may have a different micropattern form, as compared with the apparatus 100 for separating a target object described above referring to Fig. 1A. Those other than said difference or features generated from said difference may have the properties of the apparatus 100 for separating a target object described above referring to Fig. 1.

Figs. 1A to 1C illustrate the apparatus 100 for separating a target object comprising a linear micropattern. Figs. 2A to 2C illustrate the apparatus 200 for separating a target object comprising a curved micropattern and a highway channel 250. However, the apparatus for separating a target object is not limited thereto, and any apparatus for separating a target object may comprise at least one of a linear micropattern, a curved micropattern and a highway channel.

In an embodiment, the apparatus 200 for separating a target object may comprise an inlet 210 through which a fluid including microparticles is injected, an injection unit 215 which is a fluid flow passage around the inlet 210, a passage unit 220 for concentrating a target object in a predetermined direction to flow while the injected fluid flows, a target object obtaining unit 230a for obtaining the target object concentrated in a predetermined direction, a non-target object discharging unit 230b, a highway channel 250, etc. In an embodiment of the present invention, one target object obtaining unit 230a is illustrated. However, this is an exemplary embodiment, and the apparatus may comprise two target object obtaining units 230a, and three or more, e.g., four to ten, microparticles obtaining units. Similarly, Fig. 2A illustrates one non-target object discharging unit 230b, but a plurality of the units may be provided. For example, the apparatus 100 for separating a target object may comprise three or more, e.g., four to thirty, more specifically eight to fifteen, non-target object discharging units. The number of each of target object obtaining units and non-target object discharging units may be the same or different according to the target object.

In an embodiment, the target object obtaining unit 230a and the non-target object discharging unit 230b may be referred to as a first target object obtaining unit and a second target object acquiring unit, respectively, and a plurality of first target object obtaining units 230a may be provided and a plurality of second target object discharging units 230b may be provided. The inlet 210, the injection unit 215 and the passage unit 220 may respectively correspond to the inlet 110, the injection unit 115 and the passage unit 120 described above referring to Fig. 1A. Also, the target object obtaining unit 230a and non-target object discharging unit 230b may respectively correspond to the target object obtaining unit 130a and the non-target object discharging unit 130b described above referring to Fig. 1A. Thus, the detailed description on the inlet 210, the injection unit 215, the passage unit 220, the target object obtaining unit 230a and the non-target object discharging unit 230b is omitted. However, the micropattern of the apparatus 200 for separating a target object may be different from the micropattern of the apparatus 100 for separating a target object in terms of structure, shape, arrangement location, number, etc.

In an embodiment, the micropattern may be formed by engraved structures having a groove shape in the direction perpendicular to the main flow direction of a fluid.

In an embodiment, at least one of the injection unit 215, the passage unit 220, the target object obtaining unit 230a and the non-target object discharging unit 230b may comprise a plurality of engraved structures having a groove shape in the direction perpendicular to the main flow direction of the fluid.

In an embodiment, as the fluid injected into the apparatus 200 for separating a target object through the inlet 210 flows in the injection unit 215 and the passage unit 220, the target object may be separated intensively in a predetermined direction. The target object may be separated by a plurality of engraved structures arranged on the ceiling or bottom surface of the channel in the passage unit 220. In an embodiment, the engraved structure may be referred to as an engraved channel or groove. A process of separating a target object by the engraved structure will be described in detail referring to Figs. 7A-7C.

In an embodiment, a plurality of engraved structures may be formed being disconnected from each other. According to an embodiment, the engraved structures induce a secondary flow in a direction perpendicular to the direction in which the fluid primarily flows, to efficiently separate and concentrate microparticles.

In an embodiment, with respect to the main flow direction of the fluid, the engraved structure may have an inclination inclined to a certain direction in which microparticles are to be separated, which means that the engraved structure may include an inclined structure. The inclination may include an inclination of 45 to 135 degrees with respect to the main flow direction of the fluid. According to an embodiment, when a fluid including microparticles is injected into the apparatus 200 for separating a target object, the microparticles may move in the direction perpendicular to the inclined engraved structure to be concentrated in a predetermined direction and flow.

In an embodiment, the engraved structure may be a curved inclined structure in the longitudinal direction. For example, the micropattern formed by a plurality of engraved structures may be a curved shape. For example, the curve may include at least a portion of a circle, at least a portion of an ellipse, at least a portion of a cycloid, any curved shape, etc.

In an embodiment, a plurality of channels may be formed in the apparatus 200 for separating a target object. The channel is a passage extending from the inlet to the target object obtaining unit or non-target object discharging unit, and the number of channels may be determined according to the target object. The number of channels may be one to forty, specifically two to thirty, more specifically five to twenty. For example, regarding the number of channels from the inlet to the target object obtaining unit, the apparatus for separating leukocytes may have six channels, the apparatus for separating plasma may have six channels, and the apparatus for separating cells may have two channels. However, these are exemplary embodiments, and the apparatus 200 for separating a target object may have various numbers of channels.

In an embodiment, each channel may start from the inlet and divide into several channels in the middle of the passage extending to target object obtaining units or non-target object discharging units, and accordingly additional channels may be formed. For example, separate channels may be formed in the middle of the channel and have non-target object discharging units at the ends thereof. For example, in the case where there are eight non-target object discharging units, eight additional channels may be formed from the channel(s) starting from the inlet.

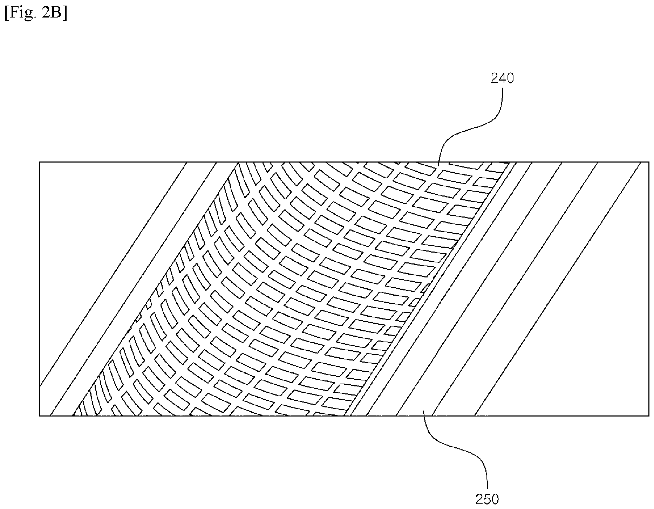

In an embodiment, the highway channel 250 may be formed in at least a portion of the area between the inlet 210 and the target object obtaining unit. In addition, no engraved structure is arranged in the highway channel 250, but a plurality of engraved structures may be arranged in an area other than the highway channel 250. The highway channel 250 will be described in detail referring to Figs. 2B, 3 and 4.

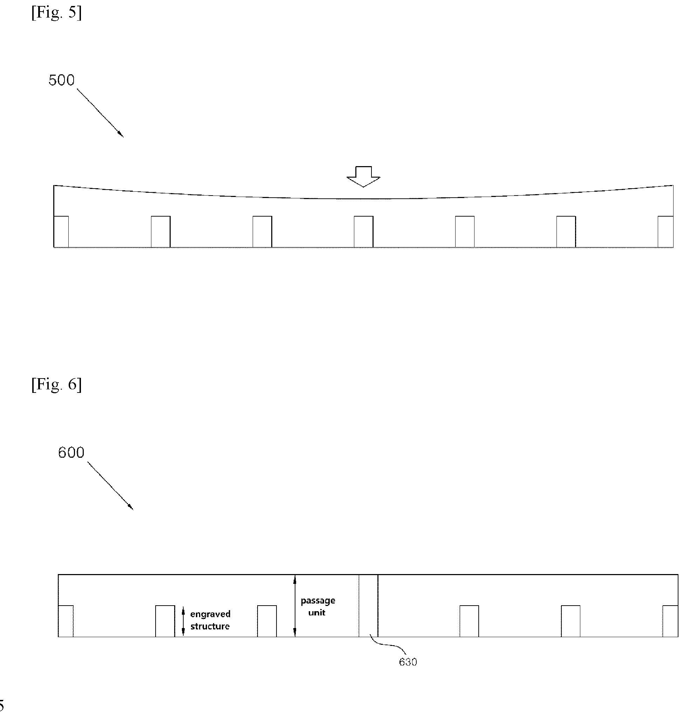

In an embodiment, at least one of the injection unit 215, the passage unit 220, the target object obtaining unit 230a and the non-target object discharging unit 230b may comprise at least one pillar structure. For example, the apparatus for separating a target object for separating leukocytes may have a pillar structure in the injection unit 215. The apparatus for separating a target object for separating plasma may have a pillar structure in the injection unit 215 and the passage unit 220. The apparatus for separating a target object for separating cells may have a pillar structure in all of the injection unit 215, the passage unit 220, the target object obtaining unit 230a and the non-target object discharging unit 230b. A plurality of pillar structures may be provided in each area. However, these are exemplary embodiments, and the apparatus for separating a target object may have several numbers of pillar structures in various areas. The pillar structure will be described in detail referring to Figs. 5 and 6.

Fig. 1A to Fig. 2C illustrate that the target object obtaining unit or non-target object discharging unit is arranged at the end of the apparatus for separating a target object. However, these are exemplary embodiments, and at least one of the target object obtaining unit and non-target object discharging unit may be located in the middle of the apparatus for separating a target object. For example, in the case where the apparatus for separating a target object is an apparatus for separating plasma, the plasma, which is the target object, obtaining unit may be located at the end of the apparatus for separating plasma, and the leukocytes or erythrocytes, which is the non-target, discharging unit may be located in the middle of the separation apparatus. Similarly, in the case where the apparatus for separating a target object is an apparatus for separating leukocytes, the leukocytes, which is the target object, obtaining unit may be located at the end of the separation apparatus, and the erythrocytes, which is the non-target, discharging unit may be located in the middle of the separation apparatus.

Fig. 2B is a partial enlarged view of the apparatus for separating a target object of Fig. 2A.

Referring to Fig. 2B, a view enlarging the portion 240 of the passage unit 220 of Fig. 2A is illustrated. In an embodiment, the passage unit may comprise a plurality of engraved structures and the highway channel 250.

In an embodiment, a plurality of engraved structures are arranged in at least a portion of the ceiling surface or bottom surface of the passage unit, so that the secondary flow may be induced in a direction perpendicular to the direction in which the fluid flows. In addition, the target object may be separated and concentrated based on the secondary flow. The engraved structure may have a predetermined inclination relative to the main flow direction of the fluid, and the inclination may be determined based on the main flow direction of the fluid and the certain direction in which microparticles are to be separated. In addition, the plurality of engraved structures may be disconnected from each other.

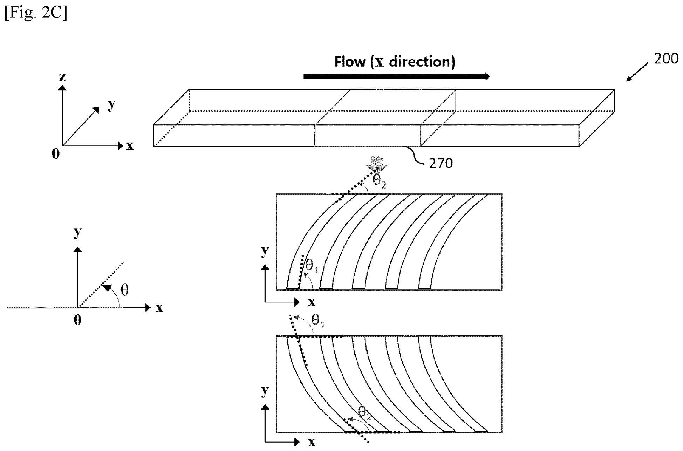

In an embodiment, the plurality of engraved structures may have a curved shape in the longitudinal direction, and the engraved structures may form a micropattern of curves. For example, as shown in Fig. 2C, a micropattern formed by the plurality of engraved structures may have an arc shape. For example, the curve may include at least a portion of a circle, at least a portion of an ellipse, at least a portion of a cycloid, any curved shape, etc.

In an embodiment, no engraved structure is arranged in the highway channel 250, but a plurality of engraved structures may be arranged only in the passage unit other than the highway channel 250. According to an embodiment, the highway channel 250 is a ditch-type channel in the depth direction, along the direction in which microparticles are concentrated. The ditch-type channel may increase the concentration efficiency of the target object and decrease loss in the opposite direction. That is, the highway channel 250 may serve as a structure to keep microparticles.

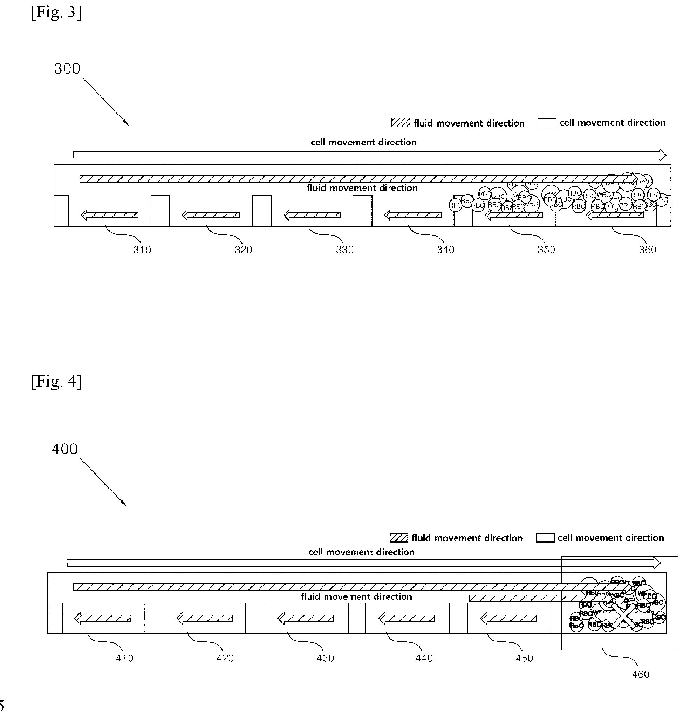

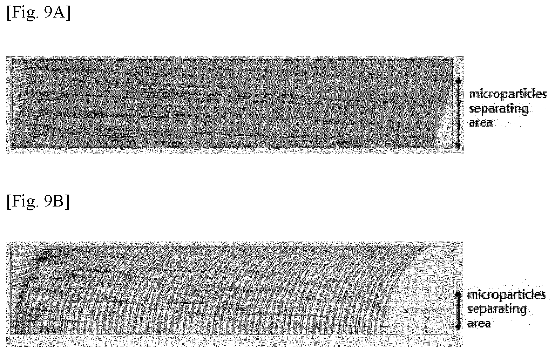

In an embodiment, the highway channel 250 may be formed in at least a portion of the area from the injection unit to the target object obtaining unit. According to an embodiment, the highway channel 250 may decrease fluid resistance and increase a local flow rate. Accordingly, a pressure is reduced, and the flow to the direction of the highway channel 250 occurs, so that the concentration efficiency of the target object may be increased. The function of the highway channel 250 will be described in more detail referring to Figs. 3 and 4. The concentration efficiency of the target object will be described in more detail referring to Figs. 9A and 9B.

Fig. 2C is a view explaining the inclination of the micropattern of the apparatus for separating a target object of Fig. 2A.

Referring to Fig. 2C, the micropattern of the apparatus 200 for separating a target object may be formed in a curved shape. In an embodiment, the curved shape may be an arc shape. Hereinafter, a micropattern will be described with an example of the portion 270 of the passage unit. When the main flow direction of the fluid is set to be on the x axis and the angle of the tangent at the starting point of the curve of the micropattern formed in the portion 270 of the passage unit relative to the x axis is set as θ₁, the angle θ₁ at the starting point of the micropattern may range from 45 degrees to 135 degrees. When the angle of the tangent at the end point of the curve of the micropattern formed in the portion 270 of the passage unit relative to the x axis is set as θ₂, the angle θ₂ at the end point of the micropattern may range from 0 degree to 75 degrees or from 105 degrees to 180 degrees. Here, the angles θ₁, θ₂ of the micropattern may be determined according to the target object.

In an embodiment, the angle θ₁ at the starting point of the micropattern may be 85 degrees and the angle θ₂ at the end point may be 45 degrees. In another embodiment, the angle θ₁ at the starting point of the micropattern may be 105 degrees and the angle θ₂ at the end point may be 135 degrees. In another embodiment, the angle θ₁ at the starting point of the micropattern in a channel of the apparatus for separating a target object may be 60 degrees and the angle θ₂ at the end point of the micropattern may be 30 degrees; and the angle θ₁ at the starting point of the micropattern in another channel may be 120 degrees and the angle θ₂ at the end point may be 150 degrees.

However, these are exemplary embodiments, and the angles θ₁ and θ₂ of the micropattern may be selected properly according to the target object.

Fig. 3 is a schematic cross-sectional view illustrating the apparatus for separating microparticles according to an embodiment of the present disclosure.

Referring to Fig. 3, the apparatus 300 for separating microparticles, more specifically the apparatus for separating cells, is described as an example of the apparatus for separating the target object. However, this is an exemplary embodiment, and the embodiments of the apparatus 300 for separating microparticles of Fig. 3 may be applied not only to cells but also to various apparatuses for separating a target object such as plasma. In an embodiment, the apparatus 300 for separating microparticles may comprise a plurality of engraved structures 310, 320, 330, 340, 350, 360. In an embodiment, a fluid injected through the injection unit of the apparatus 300 for separating microparticles may move in a certain direction while flowing and microparticles may be concentrated in a certain direction. Fig. 3 illustrates that microparticles are concentrated in the right direction as an example. A fluid including microparticles may be concentrated in the right direction based on a pattern in a certain shape formed by a plurality of engraved structures. However, reverse streamlines may occur due to the plurality of engraved structures. In this case, the concentration efficiency of microparticles may be reduced by the reverse streamlines.

In an embodiment, in order to solve the reduction of the concentration efficiency of microparticles by the reverse streamlines, an apparatus for separating microparticles comprising a highway channel which is a ditch-type channel in the depth direction may be provided on the side where microparticles are concentrated (e.g., right side in Fig. 3).

Fig. 4 is a schematic cross-sectional view illustrating the apparatus for separating microparticles comprising a highway channel according to an embodiment of the present disclosure.

Referring to Fig. 4, the apparatus 400 for separating microparticles, more specifically the apparatus for separating cells, is described as an example of the apparatus for separating a target object. However, this is an exemplary embodiment, and the embodiments of the apparatus 400 for separating microparticles of Fig. 4 may be applied to various apparatuses for separating a target object which is not only cells but also plasma. In an embodiment, the apparatus 400 for separating microparticles may comprise a plurality of engraved structures 410, 420, 430, 440, 450 and a highway channel 460. In an embodiment, no engraved structure may be arranged in the highway channel 460. In addition, the highway channel 460 may be formed as a ditch-type channel in the depth direction, on the side where microparticles are concentrated. For example, the highway channel 460 may be formed on the right side of the passage unit in Fig. 4. According to an embodiment, as no inclined structure which forms an engraved structure is arranged in the highway channel, reverse streamlines do not occur in the highway channel 460, and thus the probability that concentrated microparticles move in the reverse direction may be lowered. Accordingly, the highway channel may increase concentration efficiency and decrease loss in the opposite direction.

In an embodiment, as no inclined structure which forms an engraved structure is arranged in the highway channel 460, fluid resistance may be decreased in the highway channel more than in a unit where there are inclined structures. Accordingly, a local flow rate may be increased, and a pressure may be reduced. If a pressure is reduced, the flow of the fluid to the direction of the highway channel 460 occurs increasingly, so that the concentration efficiency of cells may be increased.

In an embodiment, the width of the highway channel may be any value ranging from 0.1% to 50% of the channel width of the apparatus for separating microparticles. For example, the width of the highway channel of the apparatus for separating plasma may range from about 20% to about 30%, e.g., about 27%, of the channel width. The width of the highway channel of the apparatus for separating cells may range from about 0.1% to 5%, e.g., about 2%, of the channel width. However, these are exemplary embodiments, and the width may be selected properly to efficiently concentrate and separate the target object.

Fig. 5 is a schematic cross-sectional view illustrating the apparatus for separating a target object in which sagging in the center of the channel occurs according to an embodiment of the present disclosure.

Referring to Fig. 5, sagging in the center of the channel may occur in the apparatus 500 for separating a target object due to thermal deformation to plastic, etc., during a process of manufacturing the apparatus 600 for separating a target object. Accordingly, the steps of the microfluid channels are changed, and the change has an impact on the separation efficiency of the target object. An embodiment of the present disclosure may provide the apparatus 600 for separating a target object comprising a pillar structure as shown in Fig. 6 in order to prevent sagging in the center of the channel. According to an embodiment of the present disclosure, channel deformation caused by sagging in the center of the channel may be prevented. Also, in addition to the sagging in the center of the channel, the damage to the target object or the adverse effect on flow may be prevented.

An embodiment of the present disclosure is to provide an apparatus for separating a target object for preventing sagging in the center of the channel.

Fig. 6 is a schematic cross-sectional view illustrating the apparatus for separating a target object which solves the sagging in the center of the channel according to an embodiment of the present disclosure.

Referring to Fig. 6, a pillar structure 630 may be installed in the apparatus 600 for separating a target object in order to prevent sagging in the center of the channel. In an embodiment, the pillar structure 630 may be arranged in at least one of the injection unit, passage unit, target object obtaining unit and non-target object discharging unit. In addition, a plurality of pillar structures 630 may be arranged. For example, in the apparatus 600 for separating the target object to separate leukocytes, the pillar structure 630 may be arranged only in the injection unit. In addition, for example, in the apparatus 600 for separating the target object to separate plasma, the pillar structure 630 may be arranged in the injection unit and passage unit. In addition, for example, in the apparatus 600 for separating the target object to separate cells, the pillar structure 630 may be arranged in all of the injection unit, passage unit and target object obtaining unit. For example, the pillar structure 630 may be arranged in the center of the channel, arranged at proper intervals, arranged between the engraved structures, or the pillar structure 630 may be installed in the center of the channel at proper intervals.

In an embodiment, the pillar structure 630 may be arranged in an area other than a plurality of engraved structures. For example, the pillar structure 630 may be arranged between at least a portion of the inclined structures which form engraved structures. In an embodiment, the pillar structure 630 is configured to be arranged not to cause hemolysis and not to affect the flow. Accordingly, the height of the pillar structure is the same as the height of the passage unit, and when the main motion direction of the fluid is on the x axis and the width direction of the channel is on the y axis, the cross section taken along the xy plane may be a circle, ellipse, streamline (e.g., ship shape), round polygon, etc. In the case where the cross section is a polygon, the cross-sectional areas at the vertices are small. Thus, when the target object or non-target object such as cells is bumped into the vertices, a great impulse occurs, which may cause damage thereto. Accordingly, in the case where the pillar structure 630 is a polygon having vertices, the cross section of the pillar structure 630 may be configured to be a round polygon with rounded vertices.

In an amendment, the height of the pillar structure 630 may be the same as the height of the passage unit. Accordingly, the pillar structure 630 may have a shape of a cylinder, cylindroid, streamlined pillar or rounded polygonal pillar.

In an embodiment, in the case where there are a plurality of pillar structures 630, the interval between the plurality of pillar structures is to be greater than the diameter of microparticles in order to prevent damage to microparticles. This is to prevent microparticle jamming and damage to microparticles such as hemolysis caused by the pillar structure.

In an embodiment, the pillar structure 630 may be made of plastic. In this case, the aspect ratio (height/length of cross section; i.e., height of passage unit/maximum length of cross section) of the pillar structure 630 is to be a certain value or less. For example, in the case where the apparatus 600 for separating a target object is manufactured using quick delivery mold (QDM), when the aspect ratio of the pillar structure 630 is greater than 3, the pillar structure may be damaged during the manufacturing process. Accordingly, the maximum length of the cross section of the structure may be determined according to the height of the passage unit. For example, in the case where the height of the passage unit is about 40 µm, the maximum length of the cross section of the structure is to be about 13 µm or more.

In an embodiment, at least one pillar structure 630 may be arranged in the point which is about 50% of the channel width from both ends of the channel, in order to prevent sagging in the center of the channel. For example, the pillar structure 630 may be arranged in the center of the channel, or the pillar structures 630 may be arranged at the points which are about 1/3, i.e., about 33%, of the channel width from both ends of the channel.

In an embodiment, in the case where there are a plurality of pillar structures 630, the interval between the pillar structures 630 is to be wider than or equal to the length of the engraved structure in order not to affect the flow of microparticles. According to the separation principle of the apparatus for separating a target object, when a sample is injected into the apparatus for separating a target object, a secondary flow (defocusing flow) may be generated along the inclined direction of the engraved structure, and a flow in the opposite direction (focusing flow) may be generated in the passage unit. Here, microparticles are assembled in one side wall by the flow in the opposite direction, so that particles may be separated. During this process, if the interval between the pillar structures is smaller than the length of the engraved structure, the flow in the direction of the engraved structure (defocusing flow) is impeded by the pillar structures 630, and accordingly, the flow in the opposite direction (focusing flow) gets weak, which may affect the separation efficiency of the apparatus for separating a target object. As such, the interval between the pillar structures 630 may be greater than the length of the engraved structure in order not to impede the secondary flow.

According to an embodiment, sagging in the center of the channel of the apparatus for separating a target object may be prevented using the pillar structure 630.

Figs. 7A to 7C are views illustrating a process of separating microparticles by a plurality of engraved structures having a groove shape according to an embodiment of the present disclosure. The engraved structures of Figs. 7A to 7C are arranged on the bottom surface of the channel in the passage unit 220.

Figs. 7A to 7C illustrate the apparatus for separating microparticles as an example of the apparatus for separating a target object, but are not limited to the apparatus for separating microparticles. The same embodiment may also be applied to the apparatus for separating plasma.

Referring to Figs. 7A to 7C, the apparatus 700 for separating microparticles may comprise a plurality of engraved structures 710, 720, 730. The engraved structures 710, 720, 730 may have a structure in a groove shape. In addition, the engraved structures 710, 720, 730 may have a shape of an inclined structure with an inclination. In an embodiment, the inclination may include an inclination of about 45 degrees to about 135 degrees with respect to the main flow direction of the fluid. For example, the apparatus for separating plasma may comprise engraved structures at an angle of about 70 to about 80 degrees, e.g., about 75 degrees. As the engraved structures 710, 720, 730 have an engraved shape, i.e., groove shape, the flow is formed in the main flow direction (bulk flow), and a secondary flow is formed in a direction perpendicular to the main flow direction (bulk flow) of the fluid as shown in Fig. 7B. Accordingly, microparticles may move transversely as shown in Fig. 7C. In addition, the engraved structures 710, 720, 730 may be formed being disconnected from each other. The engraved structures 710, 720, 730 which are formed being disconnected from each other are formed in a groove shape, which may have the effect that microparticles are assembled.

Fig. 8 is a schematic view illustrating the arrangement and alignment of a plurality of engraved structures according to an embodiment of the present disclosure.

Referring to Fig. 8, a micropattern may be formed based on a plurality of engraved structures. In Fig. 8, the length (L1) of the micropattern 800 in the longitudinal direction is defined as the length of the engraved structure, the interval (L2) between the engraved structures is defined as the disconnect interval, and the width (W) of the engraved structure in the vertical direction is defined as the width of the micropattern.

In an embodiment, the width and depth of the curved engraved structure may vary depending on the type of target object to be separated. For example, the height, width, length and interval of the engraved structures may be determined as shown in [Table 1] below. The values indicated in [Table 1] may be approximate values, and may have a deviation of e.g., ±20%, ±15%, ±10%, ±5%, or ±1%.

**[Table 1]**

| | Leukocyte separation apparatus | Plasma separation apparatus | Cell separation apparatus |
|---|---|---|---|
| Height (µm) | 20 | 10 | 20 |
| Width (µm) | 20 | 10 | 20 |
| Length (µm) | 120 | 50 | 200 |
| Interval (µm) | 20 | 10 | 30 |

In an embodiment, the height, width and interval of the engraved structures may be manufactured to be 0.5 times to 3 times the diameter of microparticles based on the fact that microparticles are 5 to 20 µm.

In an embodiment, the length of the curved engraved structure may be manufactured to be 4 times to 30 times the width of the engraved structure, and the disconnect interval of the curved engraved structures may be manufactured to be 0.1 times to 5 times the width of the micropattern.

Fig. 9A is a drawing simulating cell separation performance obtained from the micropattern having a linear shape according to an embodiment of the present disclosure. Fig. 9B is a drawing simulating cell separation performance obtained from the micropattern having a curved shape according to an embodiment of the present disclosure.

Figs. 9A and 9B show the difference in separation area of microparticles according to the shape of the micropattern of inclined structures.

Fig. 9A illustrates the linear micropattern, and Fig. 9B illustrates the curved micropattern. As a result of comparing microparticle separation performance between the linear micropattern and the curved micropattern using computational fluid dynamics (CFD), it was demonstrated that the curved micropattern is better in terms of separation performance because it has a narrower microparticle separation area.

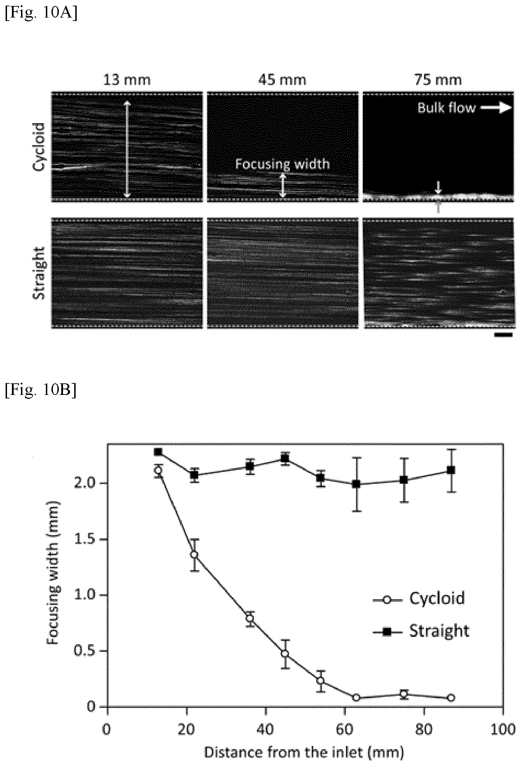

Figs. 10A and 10B are drawings showing the experimental results of cell separation performance obtained from the micropattern having a curved shape according to an embodiment of the present disclosure.

Figs. 10A and 10B show the experimental results using real cells. Fig. 10A is an image taken by a fluorescence microscope showing how many cells are concentrated at the points which are 13 mm, 45 mm and 75 mm away from the injection unit, while allowing cells stained with fluorescence to flow in the microfluidic channel having a micropattern. The top of Fig. 10A shows cell concentration in the microfluidic channel having a curved micropattern (cycloid). The bottom of Fig. 10A shows cell concentration in the microfluidic channel having a linear micropattern (straight).

Referring to Fig. 10B, it can be understood that the curved micropattern is excellent in separation performance as compared with the linear micropattern because the width in which cells are separated and concentrated is gradually decreased in the curved micropattern along the micropattern passing length.

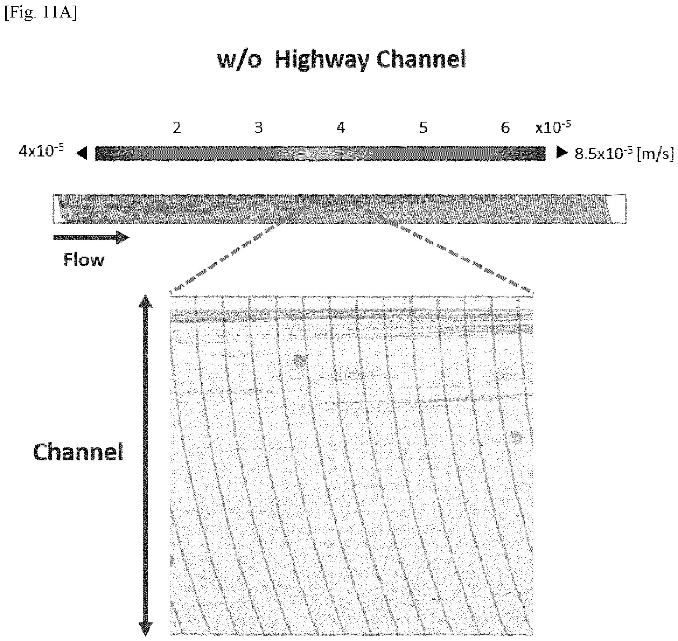

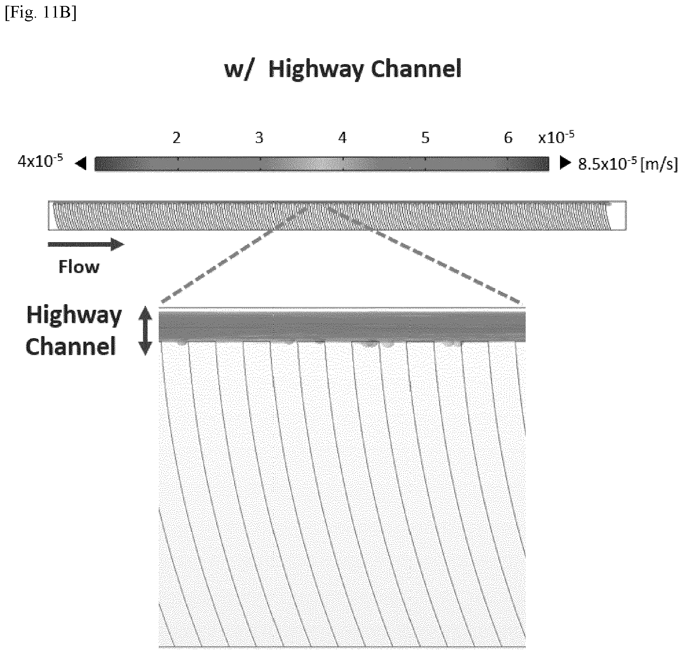

Fig. 11A is a view showing the separation performance of a target object in the case where there is no highway channel according to an embodiment of the present disclosure. Fig. 11B is a view showing the separation performance of a target object in the case where there is a highway channel according to an embodiment of the present disclosure.

Referring to Figs. 11A and 11B, the colors may indicate the trajectory along which the target object flows. The faster speed is marked in red (right in the drawing), and the slower speed is marked in blue (left in the drawing). That is, it can be confirmed that the target objects flow at a faster speed through the highway channel in Fig. 11B than in Fig. 11A. Thus, the highway channel may increase a local flow rate, and may improve the concentration efficiency of microparticles.

The foregoing description of the present disclosure is only for illustration, and those having ordinary knowledge in the art to which the present disclosure pertains will understand that the present disclosure may be easily modified in other specific forms without changing the technical spirit or essential features of the present disclosure. Therefore, it should be construed that the embodiments described above are exemplary in all aspects and not restrictive. For example, each element described as a singular form may be implemented in a distributed manner, and similarly, elements described as distributed may be implemented in a combined form.

The scope of the present disclosure is shown by the following claims rather than the above description, and all changes or modifications derived from the meaning and scope of the claims and their equivalents should be construed as being included in the scope of the present disclosure.

## Claims

1. An apparatus for separating a target object, comprising:
an injection unit into which a fluid including microparticles is injected;
a passage unit allowing a flow of the target object to be concentrated in a predetermined direction during a process of the injected fluid flowing, the passage unit comprising a plurality of engraved structures having a groove shape in a direction perpendicular to a main flow direction of the fluid; and
a target object obtaining unit for obtaining the target object concentrated in the predetermined direction,
wherein at least one of the injection unit, the passage unit or the target object obtaining unit comprise a pillar structure arranged in an area other than the plurality of engraved structures.

2. The apparatus of claim 1, further comprising a non-target object discharging unit.

3. The apparatus of claim 1, wherein the target object is concentrated in a predetermined direction by a secondary flow generated in a direction perpendicular to the main flow direction of the fluid by the plurality of engraved structures.

4. The apparatus of claim 1, comprising a highway channel extending from at least a portion of the area between the injection unit and the target object obtaining unit.

5. The apparatus of claim 4, wherein the plurality of engraved structures are arranged in the passage unit other than the highway channel.

6. The apparatus of claim 4, wherein the highway channel is formed as a ditch-type channel in a depth direction on one side of the passage unit corresponding to a direction in which the microparticles are concentrated.

7. The apparatus of claim 4, wherein the highway channel has a width of 0.1% to 50% of the width of the passage unit.

8. The apparatus of claim 1, wherein a micropattern formed by the plurality of engraved structures has a curved shape.

9. The apparatus of claim 1, wherein the plurality of engraved structures are arranged on a bottom surface or a ceiling surface of the apparatus for separating microparticles, and the plurality of engraved structures are formed being disconnected from each other.

10. The apparatus of claim 1, wherein the plurality of engraved structures form a linear micropattern, and the linear micropattern has an angle of 45 to 135 degrees with respect to the main flow direction of the fluid.

11. The apparatus of claim 1, wherein the plurality of engraved structures form a curved micropattern formed from a first point, which is a starting point, to a second point, which is an end point, and a tangent of the first point has an angle of 45 to 135 degrees with respect to the main flow direction of the fluid, and the tangent of the second point has an angle of 0 to 75 degrees or 105 to 180 degrees with respect to the main flow direction of the fluid.

12. The apparatus of claim 2,
wherein when the target object is a leukocyte, erythrocytes are obtained in the non-target object discharging unit,
wherein when the target object is plasma, blood cells are obtained from the non-target object discharging unit, and
wherein when the target object is a cell, a culture medium from which the cell is removed is obtained in the non-target object discharging unit.

13. The apparatus of claim 1,
wherein a height of the pillar structure corresponds to the height of the passage unit,
wherein the pillar structure has a pillar shape having a cross section of a circle, an ellipse, a streamline or a round polygon, and
wherein a maximum length of a cross-section of the pillar shape is determined so that (the height of the passage unit)/(the maximum length of the cross-section) does not exceed a certain value.

14. The apparatus of claim 1, wherein when there are a plurality of pillar structures, a distance between the plurality of pillar structures is wider than or equal to a diameter of a microparticle to be obtained or a length of the engraved structure.

15. A method for separating a target object from a fluid using an apparatus for separating the target object, comprising:
injecting a fluid including microparticles into an injection unit; and
obtaining, at the target object obtaining unit, the target object concentrated in a predetermined direction during a process of flowing the fluid injected into a passage unit,
wherein the passage unit comprises a plurality of engraved structures having a groove shape in a direction perpendicular to a main flow direction of the fluid, and
wherein at least one of the injection unit, the passage unit or the target object obtaining unit comprise a pillar structure arranged in an area other than the plurality of engraved structures.

16. The method of claim 15, further comprising:
obtaining a non-target object from a non-target object discharging unit.

17. The method of claim 15, wherein the target object is concentrated in a predetermined direction by a flow generated in a direction perpendicular to the main flow direction of the fluid by the plurality of engraved structures.

18. The method of claim 15, wherein the apparatus for separating the target object comprises a highway channel extending from at least a portion of the area between the injection unit and the target object obtaining unit.
